# EUROPEAN PATENT SPECIFICATION

(11) **EP 1 397 093 B1**
(45) Date of publication and mention of the grant of the patent: **11.10.2006**
(21) Application number: 01939563.1
(22) Date of filing: 25.05.2001
(51) Int. Cl.: A61F 2/28, A61F 2/08

(54) **LIGAMENT SHIM**
BÄNDER-UNTERLEGSCHEIBE
RONDELLE DE CALAGE POUR LIGAMENT

(43) Date of publication of application: 17.03.2004
(73) Proprietor: Sklar, Joseph H., Longmeadow, MA 01106 (US)
(72) Inventor: Sklar, Joseph H., Longmeadow, MA 01106 (US)
(74) Representative: Babeluk, Michael
(86) International application number: PCT/US2001/017205
(87) International publication number: WO 2002/096323

(56) References cited:
- EP-A- 0 306 018
- WO-A-00/18332
- WO-A-98/22047
- US-A- 5 632 748
- US-A- 5 876 455
- US-A- 5 899 938
- US-A- 5 931 869
- US-A- 5 961 520
- US-A- 6 001 100
- US-B1- 6 267 767

## Description

The invention relates to a ligament shim according to the preamble of claim 1.

A ligament is a piece of fibrous tissue which connects one bone to another.

Ligaments are frequently damaged (e.g., detached or torn or ruptured, etc.) as the result of injury and/or accident. A damaged ligament can impede proper motion of a joint and cause pain.

Various procedures have been developed to repair or replace a damaged ligament. The specific procedures used depend on the particular ligament which is to be restored and the extent of the damage.

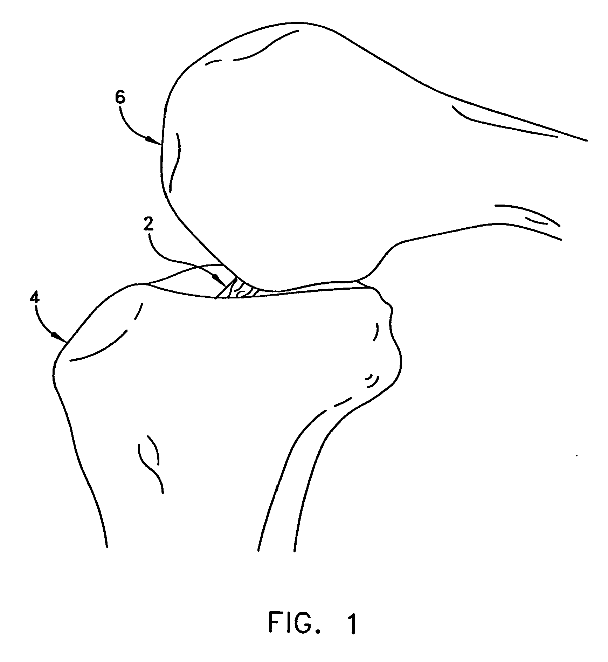

One ligament which is frequently damaged as the result of injury and/or accident is the anterior cruciate ligament (ACL). The ACL 2 extends between the top of the tibia 4 and the bottom of the femur 6 (Fig. 1). A damaged ACL can cause instability of the knee joint and cause substantial pain and arthritis.

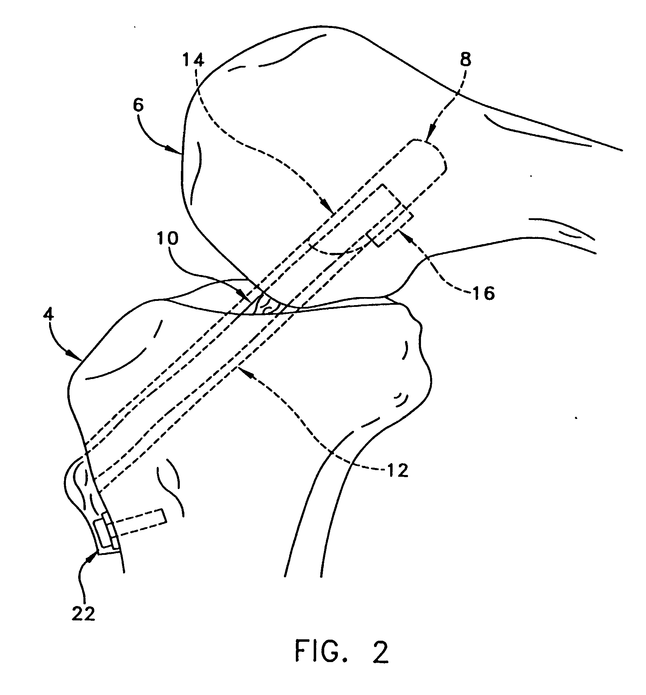

Numerous procedures have been developed to restore the ACL through a graft ligament replacement. In general, these ACL 2 replacement procedures (Fig. 2) involve drilling a bone tunnel 8 through the tibia 4 and up into the femur 6. Then a graft ligament 10, consisting of a harvested or artificial ligament or tendon(s), is passed through the tibial tunnel 12, across the interior of the joint, and up into the femoral tunnel 14. Then a distal portion of the graft ligament is secured in the femoral tunnel 14 and a proximal portion of the graft ligament is secured in the tibial tunnel 12.

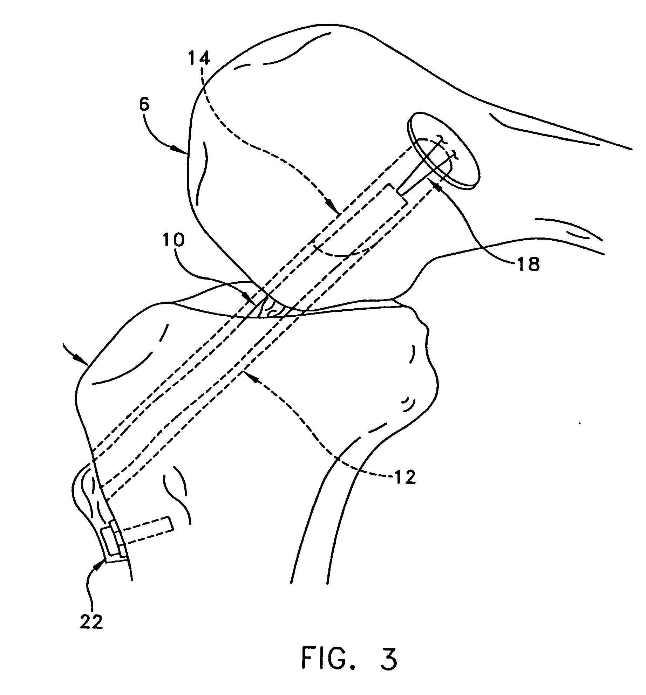

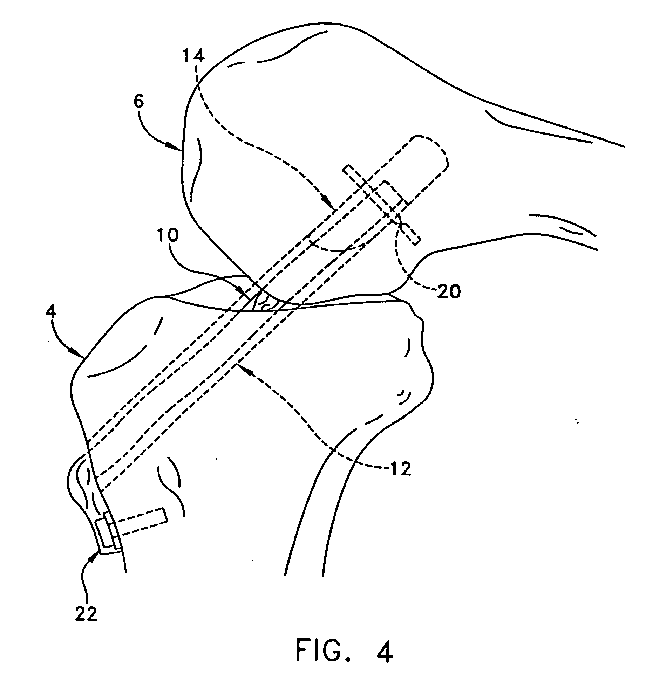

There are currently several different ways to secure a graft portion in a bone tunnel. One way is to use an interference screw 16 (Fig. 2) to aggressively wedge the graft ligament against the side wall of the bone tunnel. Another way is to suspend the graft ligament in the bone tunnel with a suture 18 (Fig. 3) or a cross-pin 20 (Fig. 4). Still another way is to pass the graft ligament completely through the bone tunnel and affix the ligament to the outside of the bone with a screw and washer arrangement 22 (Fig. 2) or a staple (not shown).

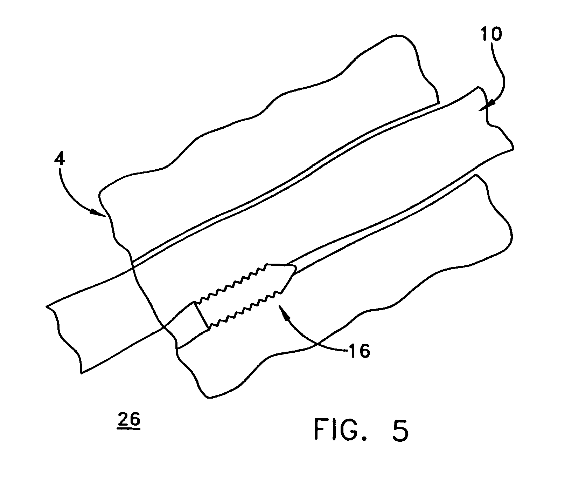

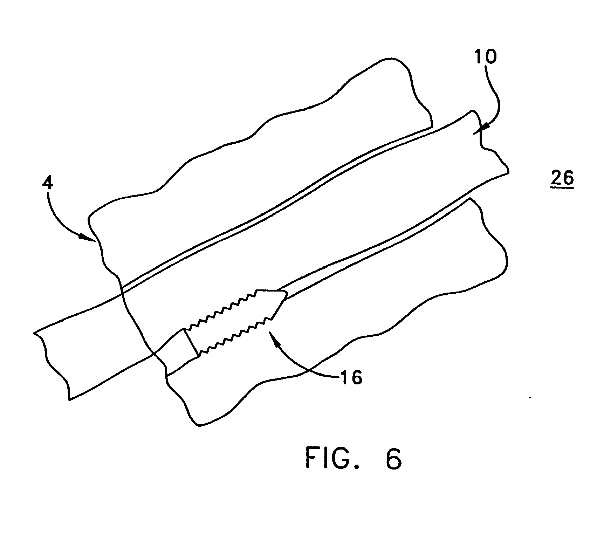

Depending on the fixation device and its manner of use, some fixation will occur at the portion of the bone tunnel nearest to the interior of the joint, and some fixation will occur intermediate the bone tunnel or adjacent to the portion of the bone tunnel farthest from the interior of the joint. For example, an interference screw 16 set into the femur 6 will typically be positioned substantially adjacent to the interior of the joint 26 (Fig. 5); however, an interference screw 16 set into the tibia 4 will frequently be positioned relatively far from the interior of the joint 26 (Fig. 6). On the other hand, suture 18 (Fig. 3) and cross-pin 20 (Fig. 4) suspensions will typically effect securing intermediate the length of the bone tunnel or at the far end of the bone tunnel, and screw and washer fixations 22 (Fig. 2) will typically effect securing relatively far from the interior of the joint 26.

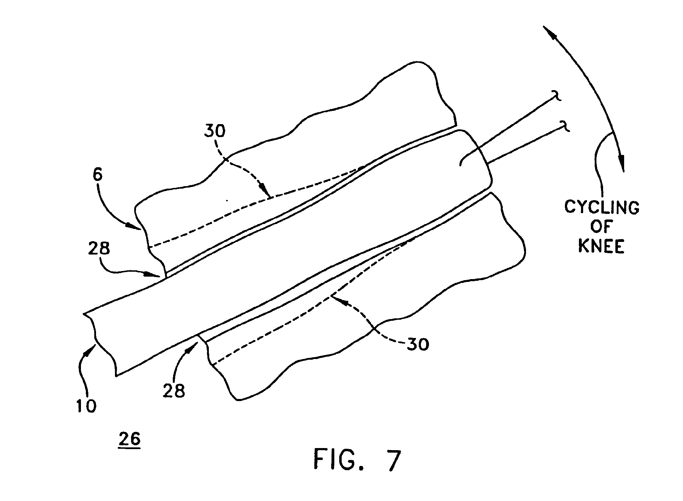

It has been observed that whenever the graft ligament is secured remote from the interior of the joint 26 (i.e., in the middle of the bone tunnel or adjacent to an outer surface of the bone), the graft ligament 10 will be relatively unsupported at the point where the ligament 10 passes from the bone tunnel into the interior of the joint. As a result, as the knee flexes back and forth through its natural range of motion (Fig. 7), the graft ligament moves about within the mouth 28 of the bone tunnel, rubbing against the walls of the bone tunnel. Over time, this can cause damage to the graft ligament and the wear down the mouth 28 of the bone tunnel, both to the serious detriment of the patient. It can also result in enlargement of the entire tunnel diameter, e.g., as show at 30. Less than a tight fit may result in incursion of synovial fluid into the tunnel, which is hypothesized to contribute to the tunnel-widening phenomenon.

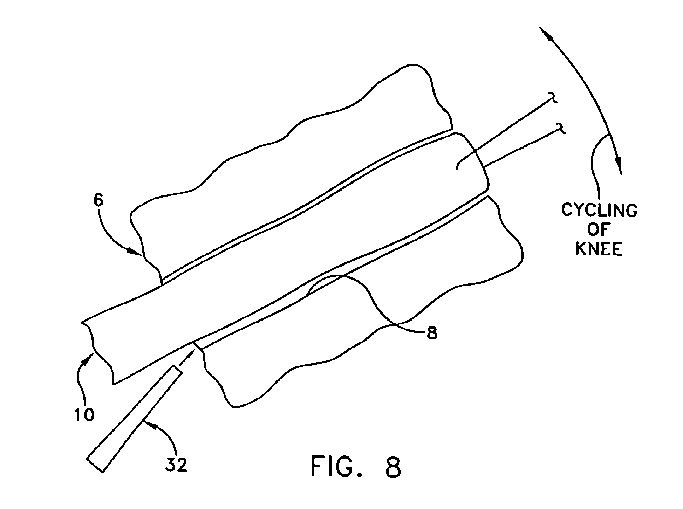

The solution to this problem is to provide a shim 32 for insertion into the mouth 28 of the bone tunnel (Fig. 8). The shim 32 is formed and sized so as to take up additional space present at the mouth 28 of the bone tunnel and, at the same time, to urge the ligament against the opposing side walls of the bone tunnel. By taking up additional space at the mouth of the bone tunnel, the aforementioned windshield wiper effect can be effectively eliminated. In addition, the entrance to the bone tunnel will be better sealed against migration of synovial fluid out of the joint and into the bone tunnel. This can be important, since incursion of synovial fluid into the bone tunnel is believed to be deleterious to the ligament reconstruction and to contribute to tunnel widening. At the same time, by urging the graft ligament 10 against the opposing side walls of the bone tunnel 8, osseo-integration between the graft ligament and the host bone will be enhanced. If desired, the shim 32 can be sized and positioned so as to force the ligament 10 against the opposing side walls of the bone tunnel 8 with substantial force so as to enhance attachment of the graft ligament 10 to the bone. However, it should also be appreciated that it is not necessary for the ligament shim 32 to force the ligament against the opposing side walls of the bone tunnel with any great force, since the primary purpose of the shim is simply to occupy excess bone tunnel space, not to compressively secure the ligament to the bone. In other words, the primary purpose of the ligament shim is to form a strategically-placed extension of the bone tunnel wall, rather to replace an interference screw.

WO 98/2204 (FEENEY et al.) shows a shim according to the preamble of claim 1. US-A-5,951,520 (BECK et al.) discloses an endosteal anchoring device for urging a ligament graft against a bone surface comprising an anchoring body, a member for resisting slippage of the anchoring body into the periphery of a bone tunnel under ligament tonsion, a member for avoiding puncturing, piercing or tearing of cross-fibers of the ligament graft and a member for urging the ligament graft flush against the inner surface of the bone tunnel for accelerated healing. WO 00/18332 (TORMALA et al.) discloses a biodegradable implant for use in the endosteal fixation of a ligament, such as the cruciate ligament, in which a ligament is attached with an anchoring device and a chute has a structural design of a smooth edge and a jagged edge, where the smooth edge lies against the ligament and the jagged side against the bone.

The ligament shim can take two basic forms a peripheral shim 34 and a centerline shim 36.

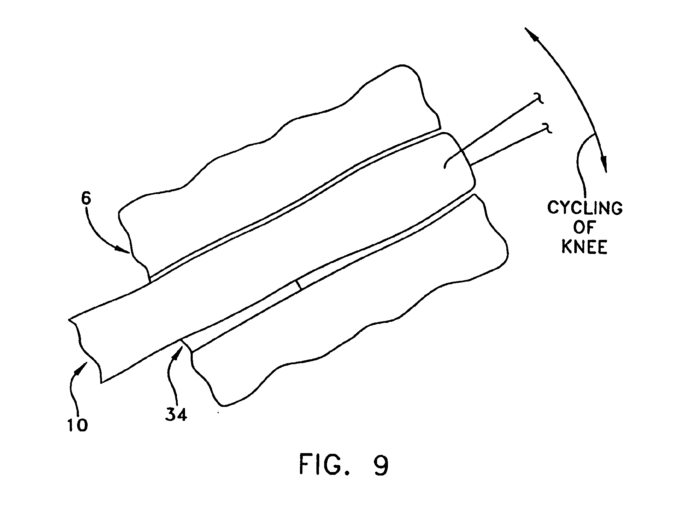

The peripheral shim 34, which does not form part of the present invention, is adapted to fit between the graft ligament 10 and a wall of the bone tunnel (Fig. 9). Thus, the shim effectively provides an extension of the bone wall which it lies against, so as to eliminate the windshield wiper effect discussed above.

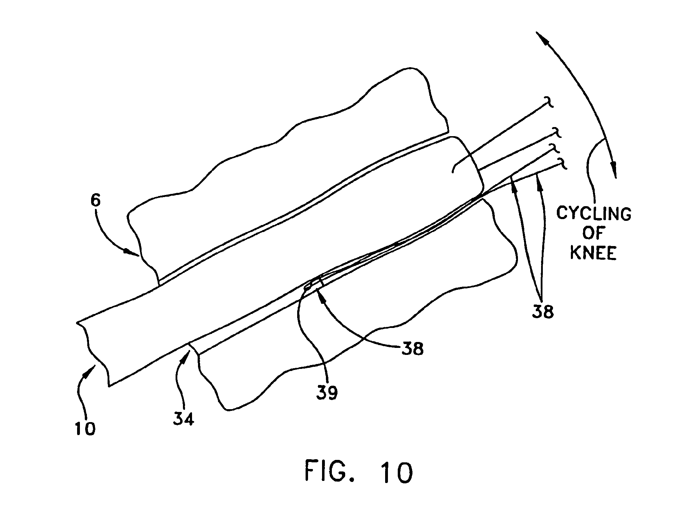

The shim 34 is intended to be held in place through a simple friction fit between the wall of the bone tunnel and the graft ligament. If desired, the shim can be tapered (Figs. 8 and 9) so as to give it a wedge-like configuration and/or the surfaces of the shim can be configured with ribs and/or roughening so as to increase friction with the adjacent anatomy. The shim can be suspended by a suture 38 which passes through a shim hole 39 (Fig. 10).

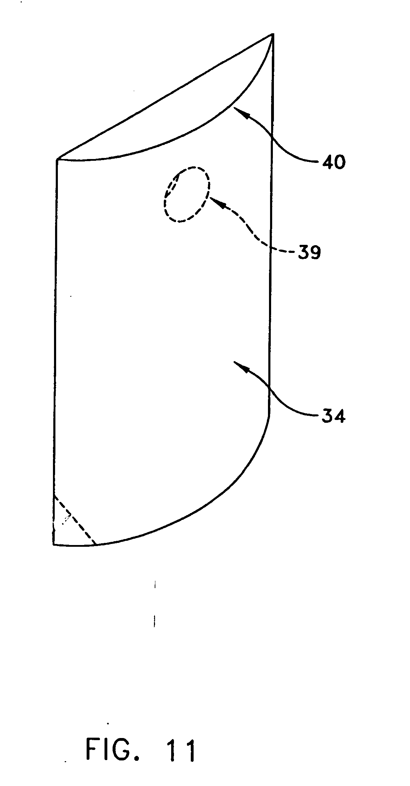

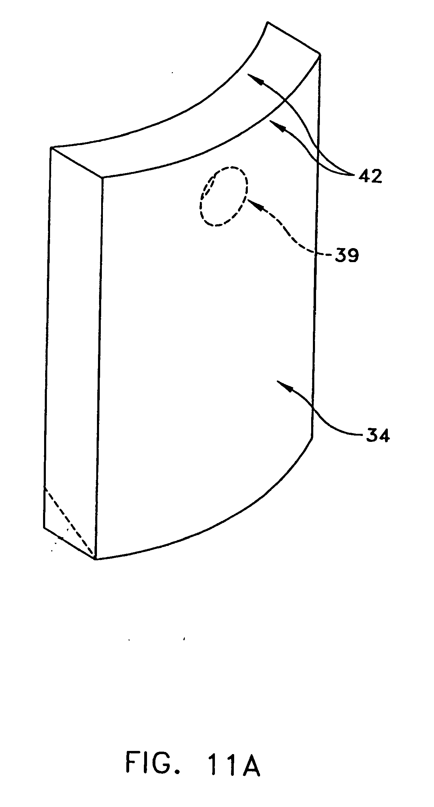

Preferably, a shim has at least its outer surface in the shape of an arc (Fig. 11), so that it can conform to the round bone tunnel wall. The shim can have both its inner and outer surfaces in the shape of an arc 42 (Fig. 11A), so that it can conform to both the round bone tunnel wall and the round graft ligament. If desired, more than one shim can be applied about the periphery of the mouth of the bone tunnel. Alternatively, a single shim can be constructed so that it covers a significant portion of the periphery of the bone tunnel wall.

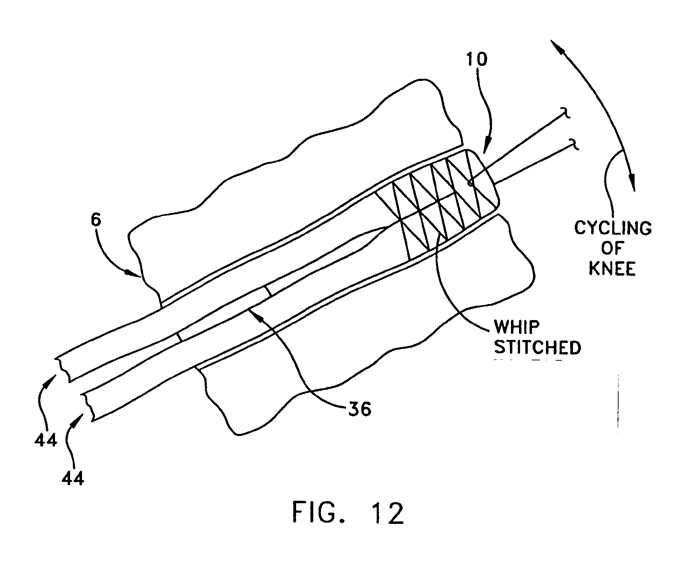

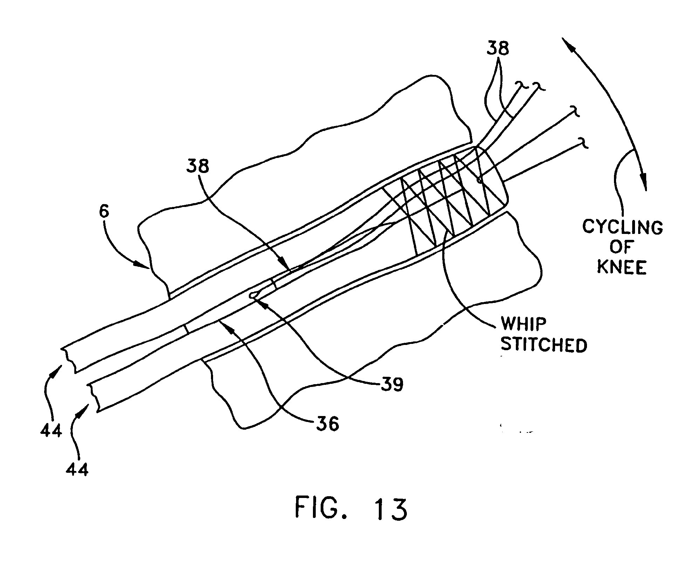

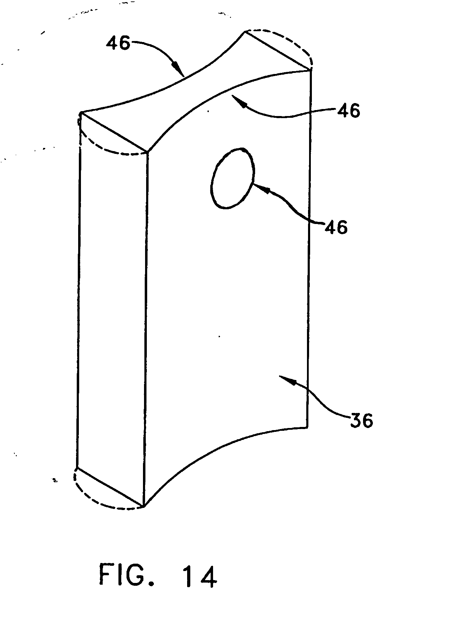

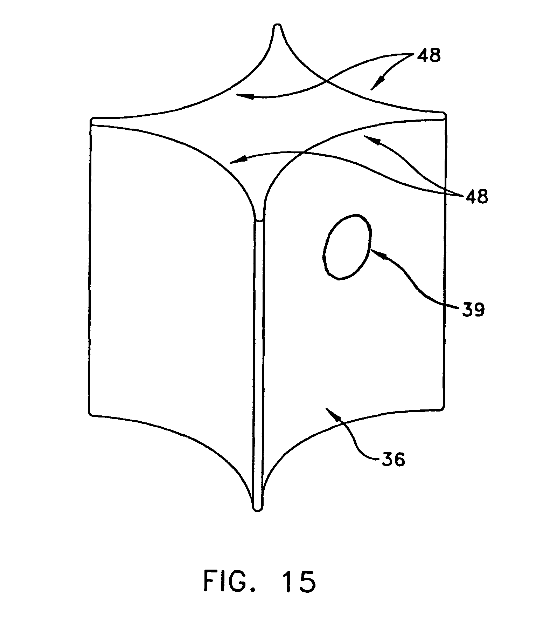

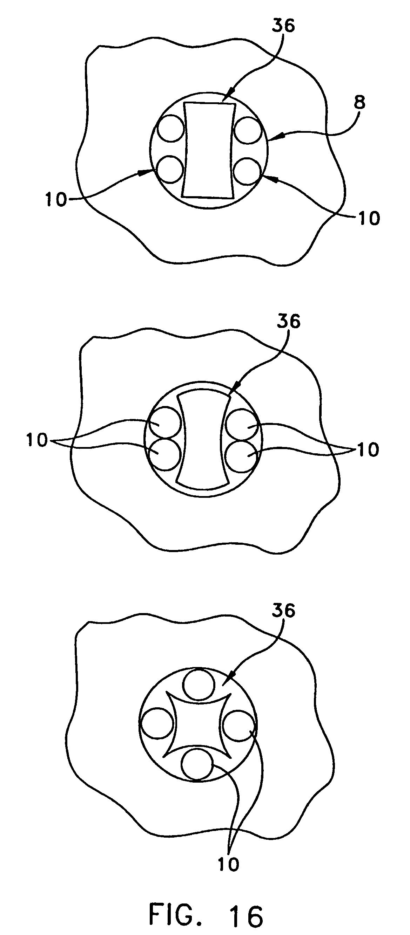

In some circumstances, the graft ligament consists of single strand of tissue (Fig. 9). In other circumstances, the graft ligament consists of two or more strands 44 of tissue which extend parallel to one another so as to collectively form the graft ligament 10 (Fig. 12). For example, suture and cross-pin suspensions are typically created by looping a long hamstring graft 44 over a suture loop or cross-pin; in this case, there are two graft ligament strands extending parallel to one another in the bone tunnel. The centerline shim 36 is adapted to fit between two such graft ligament strands 44. The centerline shim 36 can be maintained in place through a simple friction fit between the two ligament strands 44 (Fig. 12). Again, the shim can be tapered along its length so as to give it a wedge-like configuration, and/or the surfaces of the shim can be configured with ribs and/or roughening so as to increase friction with adjacent anatomy. Alternatively, and according to the present invention, the shim can be suspended by a suture 38 passing through a shim hole 39 (Fig. 13). The centerline shim according to the present invention has its two opposing surfaces in the shape of an arc 46, so that the shim can conform to the two round graft ligament strands (Fig. 14). This construction will help keep the centerline shim 36 seated between the ligament strands 44. In some cases, more than two ligament strands 44 might be used in the ligament reconstruction. For example, four ligament strands might be used in the reconstruction. In this case, the shim might comprise four arced surfaces 48 (Fig. 15). Numerous implementations of the centerline shim 36 are contemplated (Fig. 16).

Both the peripheral shim and the centerline shim also provide a benefit beyond simply curing the aforementioned windshield wiper effect. More specifically, at the same time that the shims take up excess room within the bone tunnel, they also urge the graft ligament into engagement with the walls of the bone tunnel. This urging facilitates osseo-integration between the graft ligament and the host bone, thereby improving surgical results.

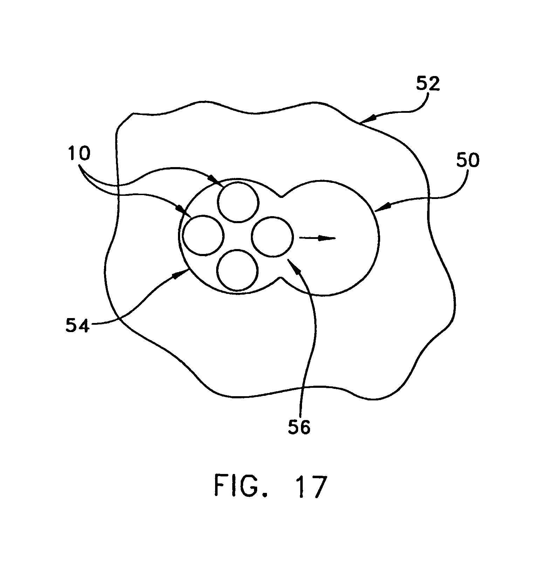

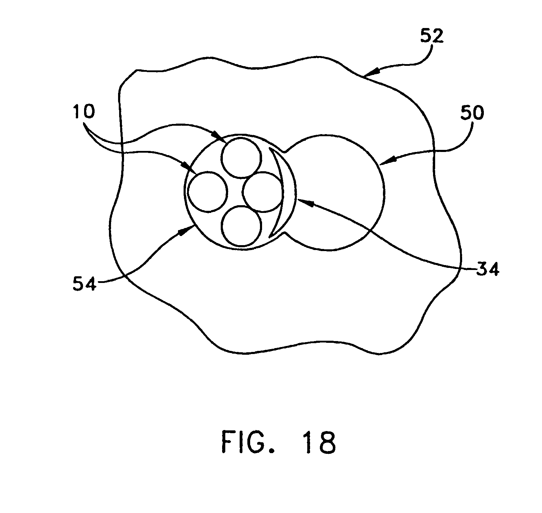

In some cases, it may be necessary to redo, or "revise", an earlier ACL reconstruction. This frequently involves forming a new bone tunnel hole adjacent to the old bone tunnel hole. If the old bone tunnel hole 50 occupied a less than ideal position in the host bone 52, it is generally desirable to place the new bone tunnel hole 54 in a better position than the old bone tunnel hole. In some circumstances, the new bone tunnel hole will be placed so close to the old bone tunnel 50 hole that the two will actually overlap (Fig. 17). In this case, there may be a danger of a graft ligament strand 10 "falling" out of the new bone tunnel hole and into the old bone tunnel hole, e.g., as show at 56. With the present invention, a peripheral shim 34 may be used (Fig. 18) so as to close off the new bone tunnel hole 54 from the old bone tunnel hole 50, so as to keep the graft ligament strand from falling into the old bone tunnel hole 50.

It should be appreciated that while the present invention has been discussed above in the context of an ACL reconstruction, it is not intended to be limited to just ACL reconstructions. The present invention will also find application in other sorts of reconstructions, e.g., other types of ligament reconstructions, etc.

## Claims

1. A ligament shim (32, 34, 36) comprising:
- a body having a first end and a second end, and an axis extending from the first end to the second end;
- the first and second ends being substantially planar and of substantially the same configuration in plan view, substantially the same size, and normal to the axis;
- the first and second ends having a length and a central width, the length being longer than the central width;
- at least a first surface (46, 48) and a second surface (46, 48) extending from the first end to the second end and substantially parallel to the axis;
wherein the first surface (46, 48) and the second surface (46, 48) are both arc-shaped and are opposed surfaces (46, 48) extending inwardly toward each other; **characterised in that** a shim hole (39) extends from the first surface (46, 48) to the second surface (46, 48).

2. The ligament shim (32, 34, 36) in accordance with claim 1 wherein said shim hole extends from a crest of the first surface (46, 48) to mid-arc portion of the second surface (46, 48).

3. The ligament shim (32, 34, 36) in accordance with claims 1 or 2 further comprising two substantially flat parallel surfaces extending from the first end to the second end.

4. The ligament shim (32, 34, 36) in accordance with one of claims 1 to 3 further comprising two outwardly rounded surfaces extending from the first end to the second end.

5. The ligament shim (32, 34, 36) in accordance with one of claims 1 to 4 further comprising two inwardly rounded surfaces (48) extending from the first end to the second end.

## Patentansprüche

1. Ligament-Klemmstück (32, 34, 36), umfassend:
- einen Körper mit einem ersten Ende und einem zweiten Ende und eine Achse, die sich von dem ersten Ende zu dem zweiten Ende erstreckt;
- wobei das erste und das zweite Ende im Wesentlichen eben und in der Draufsicht von im wesentlichen gleicher Gestalt, im Wesentlichen gleich groß und senkrecht zur Achse sind;
- wobei das erste und das zweite Ende eine Länge und eine Mittenbreite aufweisen, wobei die Länge länger als die Mittenbreite ist;
- wenigstens eine erste Fläche (46, 48) und eine zweite Fläche (46, 48), die sich von dem ersten Ende zu dem zweiten Ende erstrecken und im Wesentlichen parallel zur Achse sind;
wobei die erste Fläche (46, 48) und die zweite Fläche (46, 48) beide gekrümmt sind und gegenüberliegende Flächen (46, 48) sind, die sich einwärts zueinander hin erstrecken, **dadurch gekennzeichnet, dass** sich ein Klemmstückloch (39) von der ersten Fläche (46, 48) zur zweiten Fläche (46, 48) erstreckt.

2. Ligament-Klemmstück (32, 34, 36) nach Anspruch 1, wobei sich das Klemmstückloch von einem Scheitel der ersten Fläche (46, 48) zu einem Krümmungsmittenteil der zweiten Fläche (46, 48) erstreckt.

3. Ligament-Klemmstück (32, 34, 36) nach Anspruch 1 oder 2, das ferner zwei im Wesentlichen flache parallele Flächen umfasst, die sich von dem ersten Ende zu dem zweiten Ende erstrecken.

4. Ligament-Klemmstück (32, 34, 36) nach einem der Ansprüche 1 bis 3, das ferner zwei auswärts gerundete Flächen umfasst, die sich von dem ersten Ende zu dem zweiten Ende erstrecken.

5. Ligament-Klemmstück (32, 34, 36) nach einem der Ansprüche 1 bis 4, das ferner zwei einwärts gerundete Flächen (48) umfasst, die sich von dem ersten Ende zu dem zweiten Ende erstrecken.

## Revendications

1. Cale pour ligament (32, 34, 36) comprenant :
- un corps comportant une première extrémité et.une deuxième extrémité, et un axe s'étendant de la première extrémité à la deuxième extrémité ;
- les première et deuxième extrémités étant sensiblement planes et sensiblement de même configuration en vue en plan, sensiblement de même taille, et normales à l'axe ;
- les première et deuxième extrémités possédant une longueur et une largeur centrale, la longueur étant supérieure à la largeur centrale ;
- au moins une première surface (46, 48) et une deuxième surface (46, 48) s'étendant de la première extrémité à la deuxième extrémité et sensiblement parallèles à l'axe ;
dans laquelle la première surface (46, 48) et la deuxième surface (46, 48) sont toutes deux en forme d'arc et sont des surfaces opposées (46, 48) s'étendant vers l'intérieur l'une vers l'autre, **caractérisée en ce qu'**un trou (39) pour ligament s'étend de la première surface (46, 48) à la deuxième surface (46, 48).

2. Cale pour ligament (32, 34, 36) selon la revendication 1, dans lequel ledit trou pour ligament s'étend d'une crête de la première surface (46, 48) à la partie de milieu d'arc de la deuxième surface (46, 48).

3. Cale pour ligament (32, 34, 36) selon les revendications 1 ou 2, comportant en outre deux surfaces parallèles sensiblement planes s'étendant de la première extrémité à la deuxième extrémité.

4. Cale pour ligament (32, 34, 36) selon l'une des revendications 1 à 3, comportant en outre deux surfaces arrondies vers l'extérieur s'étendant de la première extrémité à la deuxième extrémité.

5. Cale pour ligament (32, 34, 36) selon l'une des revendications 1 à 4, comportant en outre deux surfaces (48) arrondies vers l'intérieur s'étendant de la première extrémité à la deuxième extrémité.
